(19) Europäisches Patentamt
European Patent Office
Office européen des brevets

(11) Publication number: **0 076 515**
**B1**

(12) # EUROPEAN PATENT SPECIFICATION

(45) Date of publication of patent specification: **03.07.85**

(51) Int. Cl.⁴: **B 01 J 13/02,** A 61 K 9/52

(21) Application number: **82109196.4**

(22) Date of filing: **05.10.82**

(54) **Rapidly releasable microcapsules and method for the preparation thereof.**

(30) Priority: **05.10.81 JP 159284/81**

(43) Date of publication of application:
**13.04.83 Bulletin 83/15**

(45) Publication of the grant of the patent:
**03.07.85 Bulletin 85/27**

(84) Designated Contracting States:
**CH DE FR GB IT LI**

(56) References cited:
**GB-A-1 371 840**
**US-A-3 354 863**
**US-A-3 748 277**
**US-A-3 960 757**

**CHEMICAL ABSTRACTS, vol. 94, no. 8, February 1981, page 362, no. 52812b, Columbus Ohio (USA); C.B. LIPPOLD et al.: "Control of drug liberation from microcapsules. Part 1. Legal modification for drug transport through additive-containing lipophilic membranes"**

***The file contains technical information submitted after the application was filed and not included in this specification***

(73) Proprietor: **Tanabe Seiyaku Co., Ltd.**
**No. 21 Dosho-machi 3-chome Higashi-ku**
**Osaka-shi Osaka-fu (JP)**

(72) Inventor: **Hirata, Goichi**
**No. 13-9, Nishiyama Adachi**
**Yawata-shi Kyoto-fu (JP)**
Inventor: **Samejima, Masayoshi**
**No. 2515-23, Oaza-Aomadani**
**Minoh-shi Osaka-fu (JP)**
Inventor: **Koida, Yoshiyuki**
**No. 36-6, Amanogahara-cho 2-chome**
**Katano-shi Osaka-fu (JP)**
Inventor: **Kobayashi, Yoshinori**
**No. 4-16, Kita-Sakurazuka 2-chome**
**Toyanaka-shi Osaka-fu (JP)**
Inventor: **Kida, Akira**
**No. 4, Minamibefu 6-chome**
**Settsu-shi Osaka-fu (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

**EP 0 076 515 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a method for the preparation of novel rapidly releasable ethylcellulose microcapsules containing a core material, the coating walls of which consist essentially of ethylcellulose and a water-soluble material, which can rapidly release the active compound (the core material) in gastric and intestinal tracts.

It is known to control the release of a core material in microcapsules by thickening the coating walls of microcapsules or by forming compact coating walls and thereby decreasing the permeability thereof (cf. Tamotsu Kondo and Masumi Koishi; "Microcapsules, Process for the Preparation thereof, Their Properties and Applications", issued by Sankyo Shuppan, 1977). However, while the release of core material according to these known methods is well controlled, the release of core material is also inhibited even when the core material should be released, and hence the desired activities of the main active compounds are occasionally not obtained. Particularly, in case of pharmaceutical compounds, they are usually microencapsulated with ethylcellulose in order to mask unpleasant odor or taste thereof, but in most cases, such microcapsules show retarded release of the active ingredient in the stomach.

From this viewpoint, the present inventors have extensively studied on improvement of microcapsules, and as a result, it has been found that the desired rapidly releasable microcapsules having an excellent effect of protecting the core material and being capable of releasing easily the core material in gastric and intestinal tracts can be obtained by incorporating a water-soluble material into the ethylcellulose coating walls of microcapsules containing the core material.

An object of the present invention is to provide a process for the preparation of rapidly releasable microcapsules being capable of releasing rapidly the active component (core material) in gastric and intestinal tracts when administered. This and other objects and advantages of the present invention will be apparent to persons skilled in the art from the following description.

The above object is attained according to the invention by a method for the preparation of microcapsules by incorporating a water-soluble material into the ethylcellulose coating wall thereof, which comprises:

i) dissolving ethylcellulose in cyclohexane or a mixture of cyclohexane and n-hexane, at a temperature of 75 to 80°C,

ii) dispersing particles of a core material in the solution

iii) forming the ethylcellulose coating walls on and around the particles of the core material by cooling the dispersion to cause phase-separation of ethylcellulose,

iv) adding a water-soluble material to the dispersion,

v) further cooling the dispersion to a temperature not higher than 40°C to give stable ethylcellulose microcapsules, and

vi) recovering the thus-formed microcapsules therefrom.

The core material used in the present invention includes pharmaceutical compounds and foodstuffs which may be in the form of a solid, gel or semi-solid. The particle size of the core material is not critical but is usually in the range of about 30 to 1,000 µm, preferably about 50 to 500 µm.

Ethylcellulose used for forming microcapsule coating walls on and around the particles of the core material has preferably an ethoxy content of about 46.5 to 55 W/W% and a viscosity of about 0,003 to 0,5 Pa . s (3 to 500 cP) (the viscosity of ethylcellulose is measured in a 5 W/W% solution in toluene-ethanol (4:1) at 25°C). The ethylcellulose is usually used in an amount of about 0.01 to 10 grams per gram of the core material.

The water-soluble material to be incorporated into the ethylcellulose coating walls includes all substances which can be dissolved in water and an acidic aqueous solution, preferably be dissolved in a concentration of at least about one W/V% in water at about 20 to 40°C and an acidic aqueous solution having a pH about 1.2.

Examples of the water-soluble material are disaccharides (e.g. lactose, sucrose); sugar alcohols (e.g. mannitol); polysaccharides containing 10 W/W% or more of sulfonic group in the molecule (e.g. carrageenan); hydroxyalkylcelluloses (e.g. hydroxypropylcellulose); hydroxyalkyl alkylcelluloses (e.g. hydroxypropyl methylcellulose); organic dibasic or tribasic acids or their salts (e.g. tartaric acid, sodium fumarate, citric acid); inorganic acid salts (e.g. potassium sulfate, potassium dihydrogen phosphate, ammonium phosphate, potassium metaphosphate); amino acids or their salts (e.g. sodium glutamate, alanine, lysine hydrochloride); nucleotides or their salts (e.g. 5'-inosinic acid, sodium 5'-uridylate); polymers composed of a monomer of the formula:

$$CH_2{=}\overset{\displaystyle R\atop|}{C}{-}X$$

wherein R is hydrogen atom or methyl group, and X is carboxy group, hydroxy group, or a group of the formula:

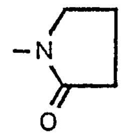 or 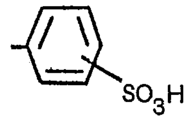

(e.g. polyvinylpyrrolidone, polyvinyl alcohol, sodium polyacrylate, polymethacrylic acid, polystyrenesulfonic acid); gum arabic; methylcellulose; polyethylene oxide; gelatin, sodium chloride; and coffee powder.

These water-soluble materials are preferably in the form of a finely-divided particle, particularly having a particle size of about 300 µm or less. The water-soluble materials are preferably used in an amount of at least about 0.01 gram, more preferably about 0.05 to 20 grams per gram of the coating wall-forming material (ethylcellulose).

In the preparation of the microcapsules of the present invention, an ethylcellulose dispersion containing a core material is firstly prepared by dispersing a core material into a solution containing ethylcellulose as the wall-forming material.

The solvent for dissolving ethylcellulose is a cyclohexane, a mixture of cyclohexane and n-hexane, which can dissolve ethylcellulose with heating (i.e. at a temperature of 70 to 80°C) but does not dissolve under cooling and further does not dissolve both of the core material and the water-soluble material; among these solvents cyclohexane is particularly suitable. Ethylcellulose is dissolved in these solvents at a temperature of 75 to 80°C. The ethylcellulose solution thus obtained has preferably a concentration of ethylcellulose of about 0.1 to 10 W/W%, more preferably about 1 to 5 W/W%. The dispersion of a core material into an ethylcellulose solution is preferably carried out with stirring at a temperature of about 75 to 80°C.

The phase-separation of ethylcellulose from the dispersion containing a core material is carried out in the presence or absence of a phase-separation-inducing agent, i.e. by either coacervation or flocculation, and optionally in the presence of a wall-forming auxiliary and/or a surfactant.

The phase-separation-inducing agent includes polyethylene, butyl rubber, polyisobutylene, and polybutadiene. The wall-forming auxiliary includes dimethylpolysiloxane, methylphenyl polysiloxane, diphenyl polysiloxane and polystyrene polydimethyl polysiloxane block copolymer. The surfactant includes an ester of $C_{12-18}$ fatty acid with sorbitan (e.g. sorbitan monolaurate, sorbitan sesquilaurate, sorbitan trilaurate, sorbitan monoleate), an ester of $C_{8-18}$ fatty acid with glycerin (e.g. glycerin monocaprylate, glycerin monolaurate, glycerin monooleate), a phospholipid (e.g. soybean phospholipid, egg-yolk phospholipid), calcium stearyl lactate, an ester of $C_{8-18}$ fatty acid with propylene glycol (e.g. propylene glycol monocaprylate, propylene glycol distearate), and an ester of $C_{12-18}$ fatty acid with sucrose (e.g. sucrose monostearate, sucrose distearate, sucrose tristearate). These additives may be added to the ethylcellulose solution when ethylcellulose is dissolved. The additives are used in a concentration of about 0.01 to 10 W/V% (phase-separation-inducing agent), about 0.01 to 10 W/V% (wall-forming auxiliary), and about 0.001 to 10 W/V% (surfactant), respectively.

The phase-separation of ethylcellulose is preferably carried out by cooling the dispersion at a rate of about 0.05 to 4°C/minute, especially 0.1 to 2°C/minute. The water-soluble material is incorporated into the ethylcellulose coating walls, in that it is added with stirring to the dispersion before cooling or during the cooling step, particularly at the stage where coating walls of ethylcellulose in the form of "gel" is formed on and around the particles of the core material and the thus-formed coating walls have still fluidity in some extent (i.e. have a viscosity of 0.01 to 5 Pa . s, especially 0.1 to 1 Pa . s). More especially, since the coating walls having a fluidity are formed on and around the core material by cooling the dispersion to about 55 to 75°C, especially about 65°C (while it may somewhat vary depending on the scale of method and cooling rate, etc.), it is preferable to add the water-soluble material to the dispersion when cooled to said temperature. The water-soluble material thus added is appropriately penetrated and dispersed into the coating walls. After adding the water-soluble material, the dispersion is further cooled to a temperature not higher than 40°C (e.g. 30 to 20°C), and thereby, the formed embryonic microcapsules are shrunk and become solid by solvent loss from the coating walls, thus giving stable ethylcellulose microcapsules.

The microcapsules thus obtained may be recovered in conventional manner, such as decantation, centrifugation, filtration and so forth, wherein the microcapsules do not adhere or coagulate each other. The microcapsules thus recovered may, if required, be washed with a solvent such as cyclohexane, petroleum ether, n-hexane, etc. and then dried in conventional manner (e.g. by a hot-air drying method or heat transfer drying method).

The microcapsules of the present invention can be applied to not only pharmaceutical medicaments but also other various substances such as veterinary drugs, foodstuffs, or the like. The pharmaceutical medicaments, to which the microcapsules of the present invention and the process for the preparation thereof can be applied, are, for example, vitamines (e.g. ascorbic acid), amino acids (e.g. potassium aspartate, magnesium aspartate), peptides (e.g. insulin), chemotherapeutics (e.g. sulfamethizole), antibiotics (e.g. benzylpenicillin potassium salt), respiratory stimulants (e.g. dimefline hydrochloride), antitussives and expectorants (e.g. tipepidine dibenzoate, bromhexine hydrochloride, trimetoquinol hydrochloride), anti-tumour agents (e.g. 5-fluorouracil, bleomycine, hydrochloride), autonomic agents (e.g. N-butylscopolammonium bromide), neuro-pyscotropic agents (e.g. calcium

N-(γ,γ-dihydroxy-β,β-dimethylbutyryl)-γ-aminobutyrate), local anesthetics (e.g. oxethazaine), muscle relaxants (e.g. phenprobamate), agents affecting digestive organs (e.g. methylmethionine sulfonium chloride, 1,1-dimethyl-5-methoxy-3-(dithien-2-ylmethylene)piperidinium bromide, precipitated calcium carbonate, trimebutine maleate), anti-histaminics (e.g. diphenhydramine hydrochloride), antidotes (e.g. D-penicillamine, diferoxamine mesylate), hypnotics and sedatives (e.g. flurazepam hydrochloride), antiepileptics (e.g. sodium valproate), antipyretics, analgesics and anti-inflammatory agents (e.g. acetylsalicylic acid, indometacin, naproxen), cardiotonics (e.g. digoxin, proscillaridin), antiarrhythmic agents (e.g. oxprenolol hydrochloride), diuretics (e.g. penfluzide), vasodilators (e.g. diltiazem hydrochloride), antilipaemics (e.g. sodium dextran sulfate), nutrients, tonics and alteratives (e.g. calcium L-aspartate), anticoagulants (e.g. heparin calcium), agents for liver disease (e.g. phosphorylcholine chloride calcium salt), antidiabetic agents (e.g. carbutamide), antihypertensives (e.g. clonidine hydrochloride), or the like.

When the microcapsules of the present invention are administered, the water-soluble material incorporated into the ethylcellulose coating walls is rapidly dissolved in water and thereby the microcapsules become porous and then water or other liquid easily penetrates inside the microcapsules, by which the active compound contained therein is rapidly released. Accordingly, in the microcapsules of the present invention, the releasing rate of the active compound can be controlled by controlling the addition amount of the water-soluble material. Besides, although the conventional microcapsules consisting of only ethylcellulose requires a fixed period of time until gastric juice penetrates into the coating walls in stomach, the microcapsules of the present invention can rapidly release the core material within a short period of time after administration, because the coating walls of the microcapsules become porous by contact with gastric juice in stomach. In addition to the above advantages, the microcapsules of the present invention have an appropriately improved ethylcellulose coating walls by incorporating a water-soluble material into the coating walls, by which the microcapsules show excellent compatibility with various carrier and also excellent fluidity and further the microcapsules can easily be tabletted without undesirable sticking or capping. The microcapsules of the present invention show also less unpleasant feeling when administered.

The present invention is illustrated by the following Experiments and Examples, wherein "part" means "part by weight" unless specified otherwise. Throughout the specification and claims, the terms "alkyl" should be interpreted as referring to alkyl having one to 4 carbon atoms.

Experiment I

Microcapsules containing trimebutine maleate (chemical name: 2-dimethylamino-2-phenylbutyl-3,4,5-trimethoxybenzoate hydrogen maleate) (a water-soluble material being incorporated into the coating walls as a release-controlling material) were prepared according to the following methods. Then, the yield of microcapsules thus obtained, the amount of the trimebutine maleate contained in the microcapsules, and the 50% release time ($T_{50}$) (i.e. a period of time which was necessary to release 50% of the active ingredient from the microcapsules) in water were examined, respectively.

Method

(i) Core material:

To a powdery mixture of trimebutine maleate (23.3 parts) and lactose (73.7 parts) was added a solution of methylcellulose (3 parts) in water (15 parts), and the mixture was kneaded in a usual manner to form granules. The granules were dried and regulated to a particle size of 105 to 350 μm.

(ii) Preparation of microcapsules:

Soybean phospholipid (0.8 g) and a silicone resin (conformable to the requirements of 4th Official Compendium of Food Additives in Japan, i.e. a mixture of dimethyl polysiloxane (viscosity: $10^{-4}$ to $11\times10^{-4}$ $m^2 . s^{-1}$ (100 to 1,100 cSt) at 25°C) and 3 to 15% by weight of silicon dioxide) (24 g) were dissolved in cyclohexane (800 ml) and thereto was added ethylcellulose (ethoxy content: 48%, viscosity: 0,1 Pa . s (100 cP) (20 g) and the mixture was dissolved by heating at 80°C. After dispersing a core material (100 g) to the solution, the dispersion was cooled with stirring at 400 r.p.m. When the temperature became to about 65°C, finely divided particles of a water-soluble material (100 g) as shown in the following Table 1 were added in order to incorporate them into the coating walls and then the mixture was cooled to room temperature. The microcapsules thus formed were separated, washed with n-hexane and dried. Said microcapsules were passed through JIS (Japanese Industrial Standard) standard sieves (500 μm) and 105 μm aperture) to give trimebutine maleate-containing microcapsules which met the requirements of "fine granules" specified in the Pharmacopoeia of Japan 10th-Edition.

As a reference, microcapsules were prepared in the same manner as described above except that no water-soluble material was added.

(iii) Results:

The results are shown in Table 1. As is clear from the results, when the water-soluble material was incorporated into the coating walls, the release of the active ingredient was promoted.

TABLE 1

| Water-soluble material | Microcapsules | | |
|---|---|---|---|
| | Yield (g) | Content of active ingredient (%) | $T_{50}$ (minute) |
| Lactose | 212 | 11.2 | 14 |
| Sucrose | 205 | 10.6 | 20 |
| Mannitol | 211 | 11.3 | 13 |
| Sodium glutamate | 203 | 10.8 | 17 |
| Citric acid | 198 | 10.7 | 15 |
| Polyvinylpyrrolidone | 200 | 10.7 | 11 |
| Polyvinyl alcohol | 203 | 10.6 | 17 |
| Methylcellulose | 199 | 10.9 | 14 |
| Hydroxypropylcellulose | 197 | 10.6 | 16 |
| Hydroxypropyl methylcellulose | 199 | 11.1 | 14 |
| Polyethylene oxide | 204 | 10.8 | 15 |
| Sodium polyacrylate | 203 | 11.2 | 13 |
| Gum arabic | 201 | 11.0 | 19 |
| Gelatine | 199 | 10.8 | 19 |
| — (reference) | 101 | 19.9 | 78 |

Experiment II

Microcapsules containing 1-methyl-5-methoxy-3-(dithien-2-ylmethylene)piperidium hydrobromide (I) (a water-soluble material being incorporated into the coating walls as a release-controlling material) were prepared, and the yield of microcapsules, the amount of the active ingredient contained in the microcapsules, and $T_{50}$ in water were examined, likewise.

Method

(i) Preparation of microcapsules:

To cyclohexane (1600 ml) were added the same silicone resin as used in Experiment I (48 g) and the same ethylcellulose as used in Experiment I (40 g), and the mixture was dissolved by heating at 80°C. After dispersing the above active compound (I) (particle size: 105—210 μm) (120 g) to the solution, the dispersion was cooled with stirring at 300 r.p.m. When the temperature became to about 65°C, finely divided particles (240 g) of a water-soluble material as shown in Table 2 in order to incorporate it into the coating walls and then the mixture was cooled to room temperature. The microcapsules thus formed were passed through JIS standard sieves (350 μm and 105 μm aperture) to give the active compound (I)-containing microcapsules which met the requirements of powders.

As a reference, microcapsules were prepared in the same manner as described above except that no water-soluble material was added.

(ii) Results:

The results are shown in Table 2. As is clear from the results, when a water-soluble material was incorporated into the coating walls, the release of the active ingredient was promoted.

TABLE 2

| Water-soluble material | Microcapsules | | |
|---|---|---|---|
| | Yield (g) | Content of active ingredient (%) | $T_{50}$ (minute) |
| Tartaric acid | 392 | 30.2 | 14 |
| Sodium fumarate | 389 | 30.4 | 31 |
| Sodium chloride | 378 | 30.8 | 21 |
| Potasium sulfate | 381 | 30.1 | 37 |
| Potassium dihydrogen phosphate | 387 | 30.5 | 23 |
| Ammonium phosphate | 391 | 30.1 | 28 |
| Alanine | 386 | 30.5 | 7 |
| Lysine hydrochloride | 384 | 30.9 | 8 |
| 5'-Inosinic acid | 383 | 30.5 | 13 |
| Sodium 5'-uridylate | 373 | 30.5 | 9 |
| Potassium metaphosphate | 393 | 30.1 | 25 |
| Coffee powder | 377 | 30.7 | 14 |
| — (reference) | 154 | 75.1 | 62 |

Example 1

Ethylcellulose (ethoxy content: 48%, viscosity: 0,1 Pa . s) (16 g) and polyisobutylene (molecular weight: about 800,000) (24 g) were dissolved in cyclohexane (800 ml) with heating. After dispersing trimebutine maleate-containing core material [prepared in the same manner as described in Experiment I-(i)] (80 g) into the solution, the dispersion was cooled with stirring at 300 r.p.m. When the temperature became to about 65°C, sucrose (80 g) was added in order to incorporate it into the coating walls and then the mixture was cooled to room temperature. The microcapsules thus formed were separated, washed with n-hexane and dried. Said microcapsules were passed through JIS standard sieves (500 µm and 105 µm aperture) to give trimebutine maleate-containing microcapsules (150 g) which met the requirements of "fine granules".

This product had a trimebutine maleate content of 12.3% and $T_{50}$ in water of 17 minutes.

Example 2

In the same manner as described in Example 1 except that polyethylene (molecular weight: 7,000) (24 g) was used instead of polyisobutylene, there were obtained trimebutine maleate-containing microcapsules (153 g) which met the requirements of "fine granules".

This product had a trimebutine maleate content of 11.9% and $T_{50}$ in water of 13 minutes.

Example 3

In the same manner as described in Example 1 except that butyl rubber [Mooney viscosity: 67 (ML-8/100°)] (24 g) was used instead of polyisobutylene, there were obtained trimebutine maleate-containing microcapsules (149 g) which met the requirements of "fine granules".

This product had a trimebutine maleate-content of 12.1% and $T_{50}$ in water of 8 minutes.

Example 4

In the same manner as described in Example 1 except that gelatine (80 g) was used instead of sucrose, there were obtained trimebutine maleate-containing microcapsules (153 g).

Example 5

In the same manner as described in Example 1 except that carrageenan (80 g) was used instead of sucrose, there were obtained trimebutine maleate-containing microcapsules (162 g).

Example 6

In the same manner as described in Example 1 except that polymethacrylic acid (80 g) was used instead of sucrose, there were obtained trimebutine maleate-containing microcapsules (154 g).

Example 7

In the same manner as described in Example 1 except that polystyrenesulfonic acid (80 g) was used instead of sucrose, there were obtained trimebutine maleate-containing microcapsules (163 g).

**Claims**

1. A method for the preparation of microcapsules by incorporating a water-soluble material into the ethylcellulose coating wall thereof, which comprises:

i) dissolving ethylcellulose in cyclohexane or a mixture of cyclohexane and n-hexane, at a temperature of 75 to 80°C,

ii) dispersing particles of a core material in the solution

iii) forming the ethylcellulose coating walls on and around the particles of the core material by cooling the dispersion to cause phase-separation of ethylcellulose,

iv) adding a water-soluble material to the dispersion,

v) further cooling the dispersion to a temperature not higher than 40°C to give stable ethylcellulose microcapsules, and

vi) recovering the thus-formed microcapsules therefrom.

2. The method according to claim 1, wherein the water-soluble material is a member selected from the group consisting of a disaccharide; a sugar alcohol; a polysaccharide containing 10 W/W% or more of sulfonic group in the molecule; a hydroxyalkylcellulose, a hydroxyalkyl alkylcellulose; an organic dibasic acid or its salt; an organic tribasic acid or its salt; an inorganic acid salt; an amino acid or its salt; a purine nucleotide or its salt, a polymer composed of a monomer of the formula:

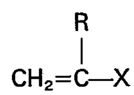

wherein R is hydrogen atom or methyl group, and X is carboxy group, hydroxy group, or a group of the formula:

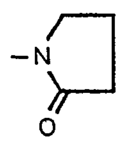

or

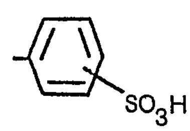

gum arabic; methylcellulose; polyethylene oxide; gelatine; sodium chloride; and coffee powder.

3. The method according to claim 1, wherein the water-soluble material is a member selected from the group consisting of lactose, sucrose, mannitol, carrageenan, hydroxypropylcellulose, hydroxypropyl methylcellulose, tartaric acid, sodium fumarate, citric acid, potassium sulfate, potassium dihydrogen phosphate, ammonium phosphate, potassium metaphosphate, sodium glutamate, alanine, lysine hydrochloride, 5'-inosinic acid, sodium 5'-uridylate, polyvinylpyrrolidone, polyvinyl alcohol, sodium polyacrylate, polymethacrylic acid, polystyrenesulfonic acid, gum arabic, methylcellulose, polyethylene oxide, gelatine, sodium chloride, and coffee powder.

4. The method according to claim 1, 2 or 3, wherein the solvent is cyclohexane.

**Patentansprüche**

1. Verfahren zur Herstellung von Mikropellets durch Inkorporieren eines wasserlöslichen Materials in die Ethylcellulosewandschicht derselben, gekennzeichnet durch:

(i) Auflösen von Ethylcellulose in Cyclohexan oder einem Gemisch aus Cyclohexan und n-Hexan bei einer Temperatur von 70 bis 80°C,

(ii) Dispergieren von Teilchen eines Kernmaterials in der Lösung,

(iii) Bildung der Ethylcellulosewandschicht auf und um die Teilchen des Kernmaterials durch Kühlen der Dispersion, wobei eine Phasentrennung der Ethylcellulose eintritt,

(iv) Zugabe eines wasserlöslichen Materials zu der Dispersion,

(v) weiteres Abkühlen der Dispersion auf eine Temperatur von nicht über 40°C unter Bildung von stabilen Ethylcellulose-Mikropellets, und

(vi) Gewinnung der auf diese Weise gebildeten Mikropellets.

2. Verfahren nach Anspruch 1, dadurch gekennzeichnet, dass das wasserlösliche Material ausgewählt wird aus der Gruppe bestehend aus einem Disaccharid, einem Zuckeralkohol, einem Polysaccharid, welches 10 G/G% oder mehr Sulfongruppen in dem Molekül enthält, einer Hydroxyalkylcellulose, einer Hydroxyalkyl-alkylcellulose, einer organischen zweibasischen Säure oder dessen Salz, einer organischen

tribasischen Säure oder dessen Salz, einem anorganischen Säuresalz, einer Aminosäure oder dessen Salz, einem Purinnucleotid oder dessen Salz, einem Polymer, das sich zusammensetzt aus einem Monomer der Formel:

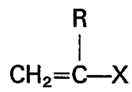

worin R ein Wasserstoffatom oder eine Methylgruppe bedeutet, und X eine Carboxygruppe, Hydroxygruppe, oder eine Gruppe der Formel:

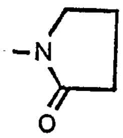
oder
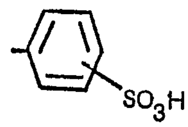

darstellt, Gummi arabicum, Methylcellulose, Polyethylenoxid, Gelatine, Natriumchlorid, und Kaffeepulver.

3. Verfahren nach Anspruch 1, dadurch gekennzeichnet, dass das wasserlösliche Material ausgewählt wird aus der Gruppe bestehend aus Lactose, Sucrose, Mannit, Karraghenan, Hydroxypropylcellulose, Hydroxypropylmethylcellulose, Weinsäure, Natriumfumarat, Zitronensäure, Kaliumsulfat, Kaliumdihydrogenphosphat, Ammoniumphosphat, Kaliummetaphosphat, Natriumglutamat, Alanin, Lysinhydrochlorid, 5'-Inosinsäure, Natrium-5'-uridylat, Polyvinylpyrrolidon, Polyvinylalkohol, Natriumpolyacrylat, Polymethacrylsäure, Polystyrolsulfonsäure, Gummi arabicum, Methylcellulose, Polyethylenoxid, Gelatine, Natriumchlorid, und Kaffeepulver.

4. Verfahren nach Anspruch 1, 2 oder 3, dadurch gekennzeichnet, dass das Lösungsmittel Cyclohexan darstellt.

**Revendications**

1. Un procédé pour la fabrication de microcapsules par incorporation d'une matière soluble dans l'eau dans leurs parois de revêtement en éthylcellulose, qui comprend les étapes suivantes:

i) dissolution d'éthylcellulose dans le cyclohexane ou dans un mélange de cyclohexane et de n-hexane, à une température de 75 à 80°C,

ii) dispersion de particules d'une matière de noyau dans la solution,

iii) formation d parois de revêtement d'éthylcellulose sur et autour des particules de la matière de noyau par refroidissement de la dispersion pour provoquer la séparation de phase de l'éthylcellulose,

iv) addition à la dispersion d'une matière soluble dans l'eau,

v) refroidissement à nouveau de la dispersion à une température ne dépassant pas 40°C pour donner des microcapsules stables d'éthylcellulose et

vi) récupération des microcapsules ainsi formées.

2. Le procédé selon la revendication 1 dans lequel la matière soluble dans l'eau est choisie parmie les disaccharides, les alcools des sucres, les polysaccharides contenant 10% en poids ou plus de groupes sulfoniques dans leurs molécules; les hydroxyalkylcelluloses, les hydroxyalkyl-alkylcelluloses; les diacides organiques ou leurs sels, les triacides organiques ou leurs sels; les sels d'acides inorganiques; les aminoacides ou leurs sels; les purines-nucléotides ou leurs sels; les polymères composés d'un monomère de formule:

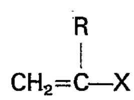

dans laquelle R est un atome d'hydrogène ou un groupe méthyle, et X est un groupe carboxy, un groupe hydroxy, ou un groupe de formule:

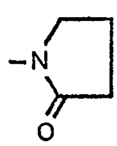
ou
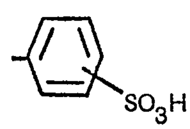

la gomme arabique; la méthylcellulose; l'oxyde de polyéthylène; la gélatine; le chlorure de sodium; et le café en poudre.

3. Le procédé selon la revendication 1, dans lequel la matière soluble dans l'eau est choisie parmi le lactose, le saccharose, le mannitol, la carraghéenine, l'hydroxypropylcellulose, l'hydroxypropylméthylcellulose, l'acide tartrique, le fumarate de sodium, l'acide citrique, le sulfate de potassium, le dihydrogénophosphate de potassium, le phosphate d'ammonium, le métaphosphate de potassium, le

glutamate de sodium, l'alanine, le chlorhydrate de lysine, l'acide 5'-inosinique, le 5'-uridylate de sodium, la polyvinylpyrrolidone, l'alcool polyvinylique, le polyacrylate de sodium, l'acide polyméthacrylique, l'acide polystyrènesulfonique, la gomme arabique, la méthylcellulose, l'oxyde de polyéthylène, la gélatine, le chlorure de sodium et la poudre de café.

4. Le procédé selon la revendication 1, 2 ou 3, dans lequel le solvant est le cyclohexane.